# EUROPEAN PATENT SPECIFICATION

(11) **EP 3 773 864 B1**
(45) Date of publication and mention of the grant of the patent: **07.06.2023**
(21) Application number: 19718892.3
(22) Date of filing: 09.04.2019
(51) Int. Cl.: A61M 39/16, A61M 39/20, A61M 5/31

(54) **UNIVERSAL CAP FOR MALE AND FEMALE CONNECTORS**
UNIVERSELLE KAPPE FÜR MÄNNLICHE UND WEIBLICHE KONNEKTOREN
CAPUCHON UNIVERSEL POUR CONNECTEURS HOMME ET FEMELLE

(30) Priority: 10.04.2018 US 201862655477 P
(43) Date of publication of application: 17.02.2021
(73) Proprietor: Becton, Dickinson and Company, Franklin Lakes, New Jersey 07417 (US)
(72) Inventor: MARICI, Paul, Piscataway, New Jersey 08854 (US)
(74) Representative: dompatent von Kreisler Selting Werner - Partnerschaft von Patent- und Rechtsanwälten mbB
(86) International application number: PCT/US2019/026480
(87) International publication number: WO 2019/199745

(56) References cited:
- EP-A1- 2 606 930
- US-A1- 2016 045 629
- US-A1- 2017 232 121

## Description

### TECHNICAL FIELD

The present disclosure generally relates to a device for disinfecting and sterilizing access ports with, e.g., male and female luer fitting, and, in particular, to disinfecting and sterilizing devices capable of accommodating multiple types of connectors.

### BACKGROUND

Vascular access devices (VAD's) are commonly used therapeutic devices and include intravenous (IV) catheters. There are two general classifications of VAD's, peripheral catheters and central venous catheters. Bacteria and other microorganisms may gain entry into a patient's vascular system from access hubs and ports/valves upon connection to the VAD to deliver the fluid or pharmaceutical. Each access hub (or port/valve or connection) is associated with some risk of transmitting a catheter related bloodstream infection (CRBSI), which can be costly and potentially lethal.

In order to decrease CRBSI cases and to ensure VAD's are used and maintained correctly, standards of practice have been developed, which include disinfecting and cleaning procedures.

Disinfection caps have been added to the Society for Healthcare Epidemiology of America (SHEA) guidelines and early indications are that caps will also be incorporated into the 2016 Infusion Nurses Standards (INS) guidelines.

In developed markets, when utilizing an IV catheter, a needleless connector will typically be used to close off the system and then subsequently accessed to administer medication or other necessary fluids via the catheter to the patient. INS Standards of Practice recommend the use of a needleless connector and state that it should be "consistently and thoroughly disinfected using alcohol, tincture of iodine or chlorhexidine gluconate/alcohol combination prior to each access." The disinfection of the needleless connector is ultimately intended to aid in the reduction of bacteria that could be living on the surface and possibly lead to a variety of catheter related complications including CRBSI. Nurses will typically utilize a 70% isopropyl alcohol (IPA) pad to complete this disinfection task by doing what is known as "scrubbing the hub." However, compliance to this practice is typically very low. In addition to a lack of compliance to "scrubbing the hub", it has also been noted through clinician interviews that there is often a variation in scrub time, dry time and the number of times the needleless connector is scrubbed.

Throughout the sequence of procedures associated with the transmission of a microorganism that can cause a CRBSI, there are many risks of contact or contamination. Contamination can occur during drug mixing, attachment of a cannula, and insertion into the access hub. Because the procedure to connect to a VAD is so common and simple, the risk associated with entry into a patient's vascular system has often been overlooked. Presently, the risk to hospitals and patients is a substantial function of the diligence of the clinician performing the connection, and this diligence is largely uncontrollable.

Currently, caps for male needleless connectors, female needleless connectors, intravenous (IV), and hemodialysis lines use different designs and are, therefore, limited to the types of connectors to which the cap can be attached. Thus, prior disinfecting caps were designed to fit one type of connector only, and were specific to one particular size and/or shape of connector. Thus, there is a need for a disinfecting device capable of accommodating multiple types of connectors to streamline the disinfecting process. There is also a need for a disinfecting device capable of continuous disinfection for multiple days. US 2017/232121 discloses a disinfection device according to the state of the art.

### SUMMARY

One aspect of the present disclosure pertains to a device for connection to a medical connector. According to an exemplary embodiment of the present disclosure, a device generally comprises a cap, absorbent material, a disinfectant or an antimicrobial agent, an elastic sealing ring, a locking lid, and a peelable seal. The cap comprises an integral body, a closed end, an annular wall having a length extending from the closed end to an open end that defines a chamber containing an absorbent material and disinfectant or antimicrobial agent. The open end defines an end face and includes a peripheral ledge extending radially inward from the annular wall. The open end defines an engagement surface.

The annular wall of the cap comprises an exterior wall surface and an interior wall surface. The interior wall surface defines an opening adjacent the open end.

The elastic sealing ring comprises a dilatable opening therethrough sized and adapted to receive a male luer connector, a female luer connector, and a hemodialysis connector. The dilatable opening can be sized to frictionally engage a male luer connector. The dilatable opening has a diameter that is dilatable from an initial diameter of from about 7-9 mm to a dilated diameter of about 9-12 mm. In one or more embodiments, the peripheral ledge is sized and adapted to receive an elastic sealing ring. In one or more embodiments, the elastic sealing ring is in contact with the peripheral ledge. In one or more embodiments, the male luer connector frictionally engages the dilatable opening via a press-fit connection upon insertion into the chamber through the dilatable opening of the elastic sealing ring.

In one or more embodiments, the peripheral ledge is sized and adapted to receive the elastic sealing ring.

In one or more embodiments, the interior wall surface comprises internal threads adjacent to the closed end. The internal threads are adapted and sized to engage a female luer connector. In one or more embodiments, the internal threads partially extend along a length of the interior wall surface of the cap adjacent the closed end of the cap.

The absorbent material and the disinfectant or the antimicrobial agent contacts the male luer connector, the female luer connector and the hemodialysis connector after insertion of the connector through the dilatable opening of the elastic sealing ring.

The locking lid engages with the engagement surface and secures the elastic sealing ring to the open end. The locking lid includes an end surface.

The peelable seal can be disposed on the end face of the locking lid or on the cap to prevent the disinfectant or the antimicrobial agent from exiting the chamber.

In one or more embodiments, the female luer connector is selected from the group consisting essentially of needle-free connectors, stopcocks, and hemodialysis connectors.

In one or more embodiments, the male connector is an intravenous tubing end or stopcock.

The elastic sealing ring comprises an elastomeric material.

In one or more embodiments, the elastomeric material of the elastic sealing ring comprises a thermoplastic elastomer.

The cap and the locking lid can be made from any of a number of types of plastic materials such as polycarbonate, polypropylene, polyethylene, glycol-modified polyethylene terephthalate, acrylonitrile butadiene styrene or any other moldable plastic material used in medical devices. In one or more embodiments, the cap and the locking lid comprises a polypropylene or polyethylene material. In one or more embodiments, the exterior cap surface includes a plurality of grip members.

In one or more embodiments, the absorbent material has slits. In one or more embodiments, the absorbent material is under radial compression by the internal threads to retain the absorbent material in the chamber. In one or more embodiments, the absorbent material is retained in the chamber without radial compression by the internal threads. In one or more embodiments, the absorbent material is a nonwoven material, foam or a sponge. In a specific embodiment, the foam is a polyurethane foam.

In one or more embodiments, the disinfectant or antimicrobial agent is selected from the group consisting essentially of isopropyl alcohol, ethanol, 2-propanol, butanol, methylparaben, ethylparaben, propylparaben, propyl gallate, butylated hydroxyanisole (BHA), butylated hydroxytoluene, t-butyl-hydroquinone, chloroxylenol, chlorohexidine, chlorhexidine diacetate, chlorohexidine gluconate, povidone iodine, alcohol, dichlorobenzyl alcohol, dehydroacetic acid, hexetidine, triclosan, hydrogen peroxide, colloidal silver, benzethonium chloride, benzalkonium chloride, octenidine, antibiotic, and mixtures thereof. In a specific embodiment, the disinfectant or antimicrobial agent comprises at least one of chlorhexidine gluconate and chlorhexidine diacetate. In one or more embodiments, the disinfectant or antimicrobial agent is a fluid or a gel.

Compression of the absorbent material toward the closed end of the chamber upon connection to the female luer connector or the male luer connector allows the connector to contact the disinfectant or antimicrobial agent to disinfect the female luer connector or the male luer connector.

In one or more embodiments, the peelable seal comprises an aluminum or multi-layer polymer film peel back top. In a specific embodiment, the peelable seal is heat-sealed or induction sealed to the end face of the locking lid.

A second aspect of the present disclosure pertains to a method of disinfecting a medical connector. The method comprises connecting the device of one or more embodiments to a medical connector, wherein connecting includes engaging the interior wall surface upon insertion into the chamber such that the medical connector contacts the absorbent material and the disinfectant or antimicrobial agent.

A third aspect of the present disclosure pertains to an assembly. The assembly comprises the device of one or more embodiments connected to a medical connector. In one or more embodiments, the medical connector is selected from a male luer connector, a female luer connector, and needleless connector.

### BRIEF DESCRIPTION OF THE DRAWINGS

Figure 1 shows a perspective view of an embodiment of a device of the present disclosure;
Figure 2 shows a perspective elevation view of a device according to an embodiment of the present disclosure;
Figure 3 shows a partial sectional view of the components of a device according to an embodiment of the present disclosure taken along line 3-3 of Figure 1;
Figure 4 shows a bottom view of a component of the device of FIG. 1;
Figure 5 shows a perspective view of an exemplary elastic sealing ring of the device of FIG. 1;
Figure 6 shows a perspective view of an exemplary peelable seal of the device of FIG. 1;
Figure 7 shows a perspective view of an exemplary locking ring of the device of FIG. 1;
Figure 8 shows a bottom perspective view of an exemplary locking ring of the device of FIG. 1;
Figure 9 shows a top view of an exemplary absorbent material of the device of FIG. 1;
Figure 10 shows a perspective view of an exemplary absorbent material of the device of FIG. 1;
Figure 11 shows a perspective view of a female luer connector with septum according to the prior art;
Figure 12 shows a perspective view a female luer connector with stopcock according to the prior art;
Figure 13 shows a perspective view of a male luer connector according to the prior art;
Figure 14 shows a perspective view of a hemodialysis connector according to the prior art.

### DETAILED DESCRIPTION

Before describing several exemplary embodiments of the disclosure, it is to be understood that the disclosure is not limited to the details of construction or process steps set forth in the following description. The disclosure is capable of other embodiments and of being practiced or being carried out in various ways.

Embodiments of the disclosure pertain to a sterile, universal single-use device for connection to and disinfection of a medical connector, including male luer connectors and female luer connectors, in which the device comprises a cap, an elastic sealing ring, absorbent material, a disinfectant or the antimicrobial agent and a peelable seal. The device provides a mechanical barrier for connectors and contains an antimicrobial agent for disinfection. The device of the present disclosure allows the practitioner to streamline the disinfecting process.

With respect to terms used in this disclosure, the following definitions are provided.

As used herein, the use of "a," "an," and "the" includes the singular and plural.

As used herein, the term "catheter related bloodstream infection" or "CRBSI" refers to any infection resulting from the presence of a catheter or IV line.

As used herein, the term "Luer connector" refers to a connection collar that is the standard way of attaching syringes, catheters, hubbed needles, IV tubes, etc. to each other. The Luer connector consists of male and female interlocking tubes, slightly tapered to hold together better with even just a simple pressure/twist fit. Luer connectors can optionally include an additional outer rim of threading, allowing them to be more secure. The Luer connector male end is generally associated with a flush syringe and can interlock and connect to the female end located on the vascular access device (VAD). A Luer connector comprises a distal end, a proximal end, an irregularly shaped outer wall, a profiled center passageway for fluid communication from the chamber of the barrel of a syringe to the hub of a VAD. A Luer connector also has a distal end channel that releasably attaches the Luer connector to the hub of a VAD, and a proximal end channel that releasably attaches the Luer connector to the barrel of a syringe.

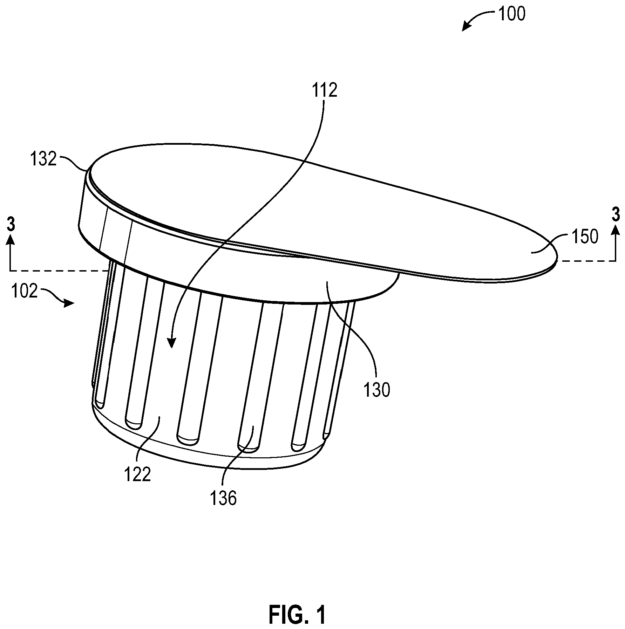

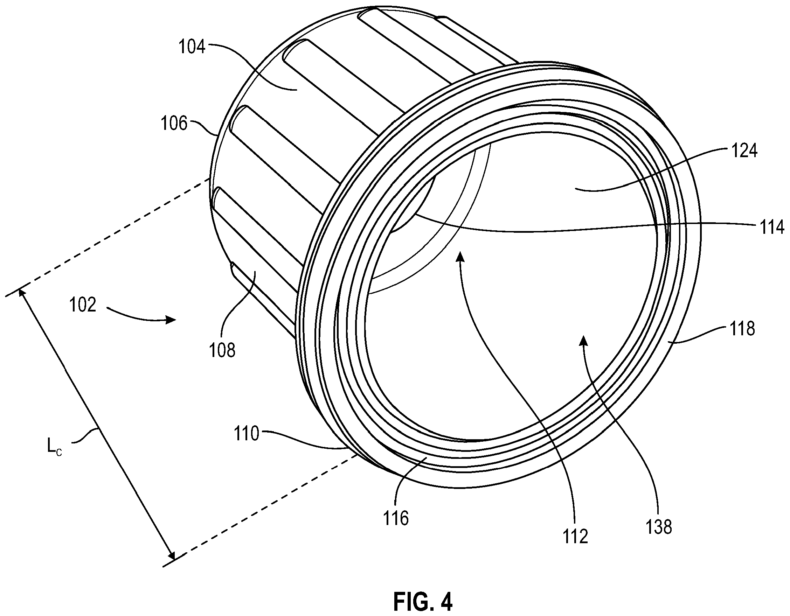

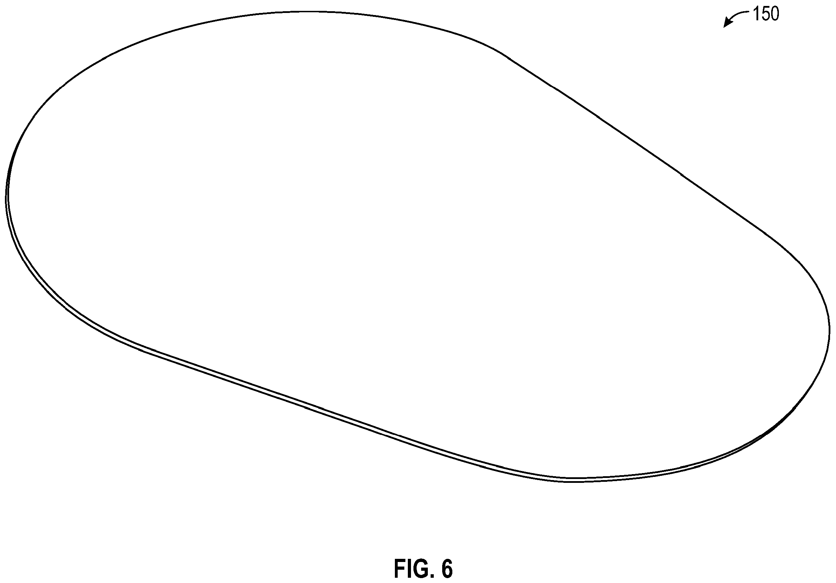

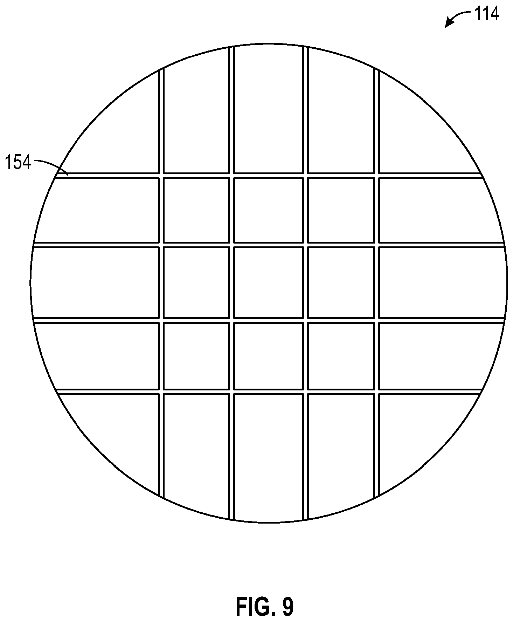

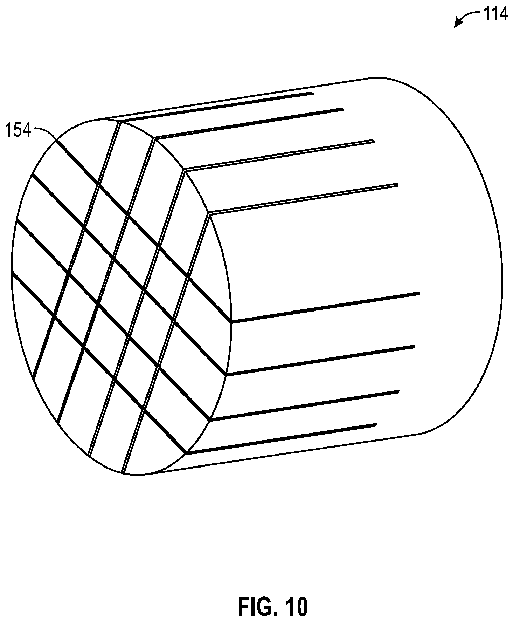

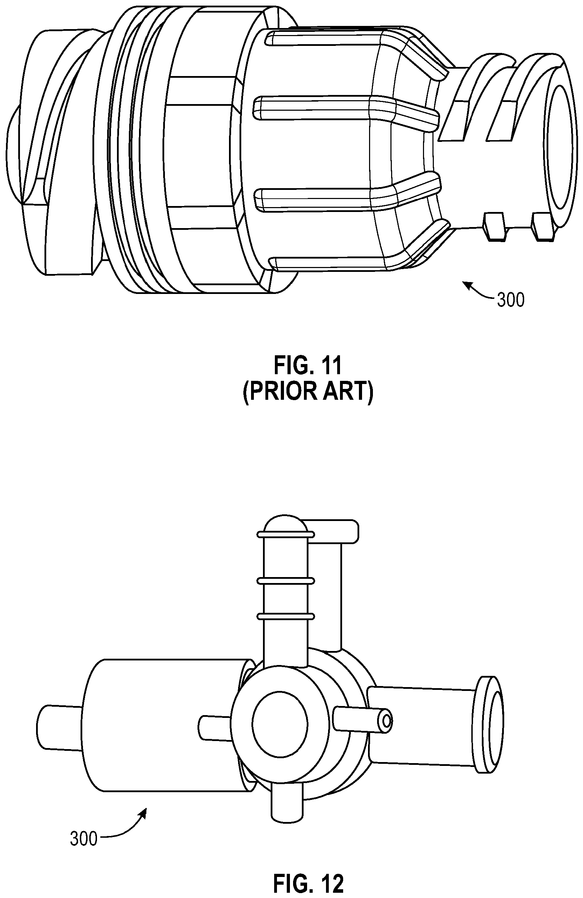

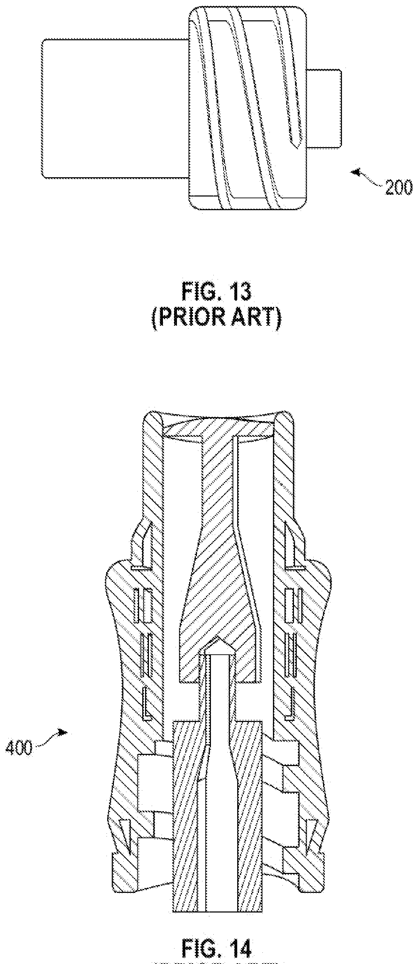

The assembled device is shown in Figures 1-3, with the components shown separately in Figures 4-10. Figures 11-14 show various connectors according to the prior art. Referring to Figures 1-3, a device 100 for connection to a medical connector according to an exemplary embodiment of the present disclosure generally comprises a cap 102, absorbent material 114, a disinfectant or an antimicrobial agent, an elastic sealing ring 126, a locking lid 130, and a peelable seal 150. Referring to Figure 4, the cap 102 comprises an integral body 104, a closed end 106, an annular wall 108 having a length L_{C} extending from the closed end 106 to an open end 110 that defines a chamber 112 containing an absorbent material 114 and disinfectant or antimicrobial agent. The open end 110 defines an end face 118 and comprises a peripheral ledge 116 extending radially inward from the annular wall 108. The open end 110 also defines an engagement surface 120.

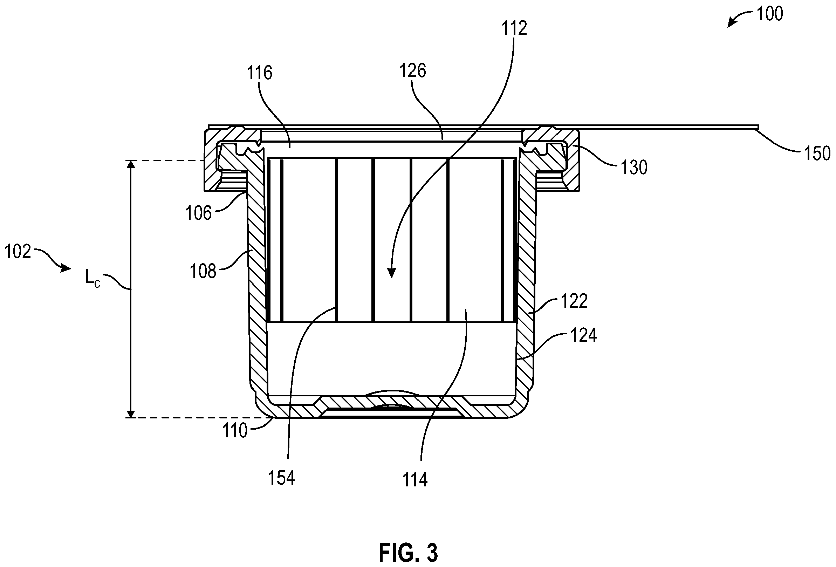

Referring to Figures 3 and 4, the annular wall 108 of the cap 102 comprises an exterior wall surface 122 and an interior wall surface 124. The interior wall surface 124 defines an opening 138 adjacent the open end 110.

Referring to Figure 3, in one or more embodiments, the elastic sealing ring 126 is in contact with the peripheral ledge 116.

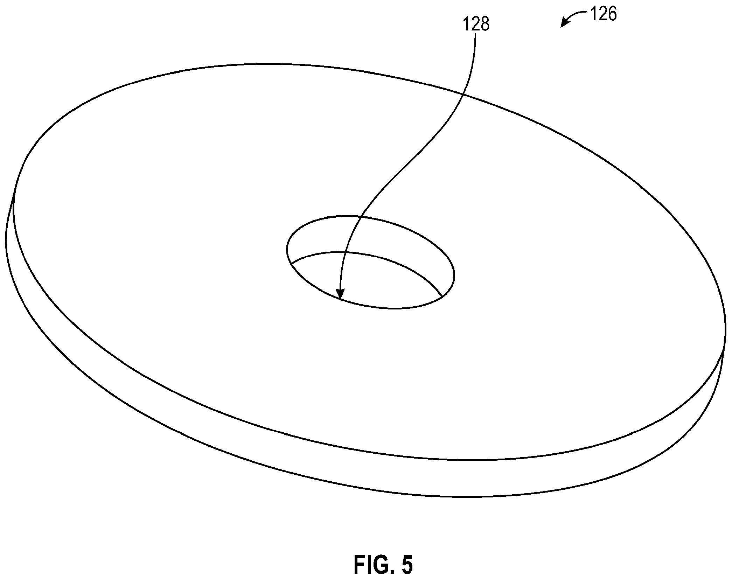

Referring to Figure 5, the elastic sealing ring 126 comprises a dilatable opening 128 therethrough sized and adapted to receive a male luer connector, a female luer connector, and a hemodialysis connector. The dilatable opening 128 can be sized to frictionally engage a male luer connector. In one or more embodiments, the dilatable opening 128 has a diameter that is dilatable from an initial diameter in a range from about 7-9 mm to a dilated diameter in a range from about 9-12 mm. In one or more embodiments, the male luer connector frictionally engages the dilatable opening 128 via a press-fit connection upon insertion into the chamber 112 through the dilatable opening 128 of the elastic sealing ring 126. In one or more embodiments, the elastic sealing ring 126 is the shape of an O-ring.

Thus, use of the device 100 requires only one single mounting movement by a user. Use of the device 100 does not activate the fluid path of a female luer connector having a septum or a hemodialysis connector having a sheath.

Referring to Figure 3, in one or more embodiments, the elastic sealing ring 126 is in contact with the peripheral ledge 116. Referring to Figures 3 and 4, in one or more embodiments, the opening 138 adjacent the open end 110 of the interior wall surface 124 is sized and adapted to receive an elastic sealing ring 126 in a press-fit connection.

In one or more embodiments, the interior wall surface 124 comprises internal threads adjacent to the closed end 106. The internal threads are adapted and sized to engage a female luer connector. In one or more embodiments, the internal threads adjacent the closed end 106 of the cap 102 partially extend along a length of the interior wall surface 124 of the cap 102.

The absorbent material 114 and the disinfectant or the antimicrobial agent contacts the male luer connector 200, the female luer connector 300, and the hemodialysis connector 400 after insertion of the connector through the dilatable opening 128 of the elastic sealing ring 126.

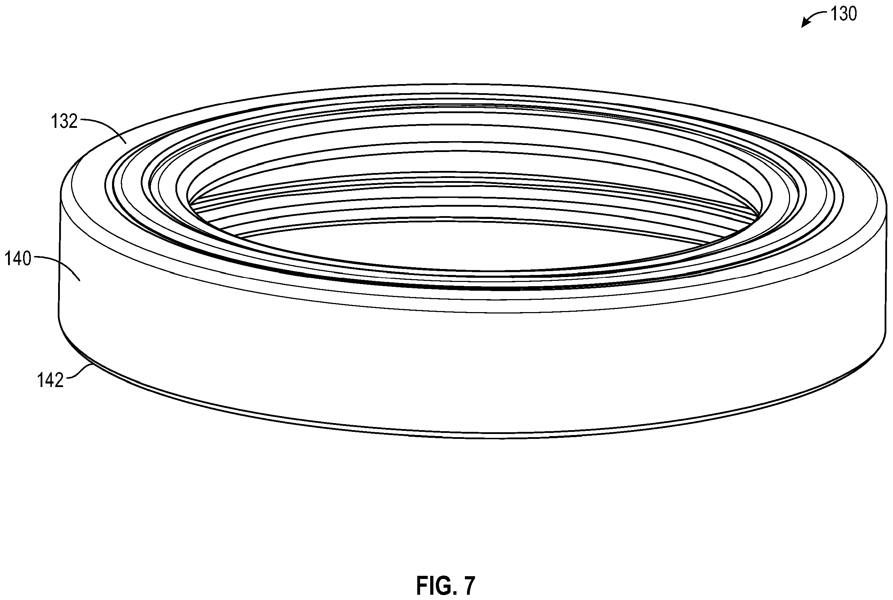

Referring to Figure 1 and to Figures 7-8, the locking lid 130 engages with the engagement surface 120 of the cap 102 and secures the elastic sealing ring 126 to the open end 110 of the cap 102. The locking lid 130 has an end surface 132, where the peelable seal 150 may be secured.

Referring to Figures 1-3 and Figure 6, in one or more embodiments, the peelable seal 150 is disposed on the end surface 132 of the locking lid 130 to prevent the disinfectant or the antimicrobial agent from exiting the chamber 112. With the absorbent material 114 properly inserted into the chamber 112 of the cap 102, the peelable seal 150 may be secured to the end surface 132 of the locking lid 130 to seal the device 100. The peelable seal 150 minimizes entry of potential particulate hazard and also provides a substantially impermeable enclosure for the device 100, provides a leak prevention and protection enclosure, protects the contents of absorbent material 114 contained within the chamber 112, and/or maintains a sealed, sterilized environment. The peelable seal 150 provides a sufficient seal at a range of temperatures, pressures, and humidity levels.

The interior wall surface 124 comprises internal threads adjacent to the closed end 106. The internal threads are adapted and sized to engage a female luer connector. The absorbent material 114 and the disinfectant or the antimicrobial agent contacts the male luer connector, the female luer connector, and the hemodialysis connector after insertion of the connector into the open end 110 of the cap 102.

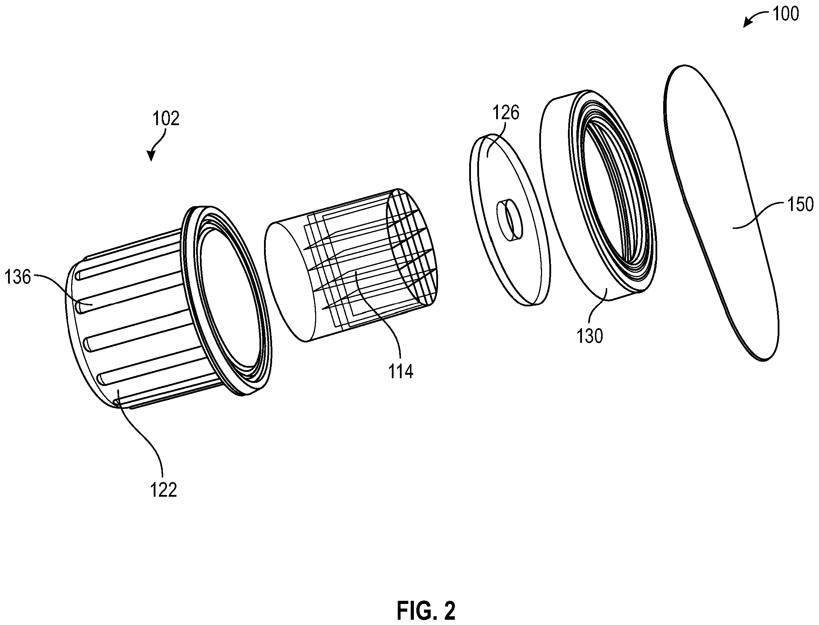

Referring to Figures 1 and 2, in one or more embodiments, the exterior wall surface 122 of the cap 102 comprises a plurality of grip members 136.

Referring to Figure 3, the cap 102 comprises an annular cap wall 108 having a cap wall length L_{C} extending from a cap closed end 106 to a cap open end 110. The cap 102 comprises an interior wall surface 124 and an exterior wall surface 122.

In one or more embodiments, the female connector may be selected from the group consisting essentially of needle-free connectors, catheter luer connectors, stopcocks, and hemodialysis connectors. In one or more embodiments, the needleless connector is selected from a Q-Syte connector, MaxPlus, MaxPlus Clear, MaxZero, UltraSite, Caresite, In Vision-Plus, Safeline, OneLink, V-Link, ClearLink, NeutraClear, Clave, MicroClave, MicroClave Clear, Neutron, NanoClave, Kendall, Nexus, InVision, Vadsite, Bionector, etc.

In one or more embodiments, the male connector may be an intravenous tubing end, a stopcock or male lock luer.

In one or more embodiments, the male luer connector rests on the peripheral ledge upon being fully inserted in into the chamber 112.

In one or more embodiments, the internal threads may be disposed adjacent the closed end 106 of the cap 102 partially extend along a length of the interior wall surface 124 of the cap 102.

In one or more embodiments, the male luer connector frictionally engages the interior wall surface 124 via a press-fit connection upon insertion into the chamber 112.

Referring to Figure 4, in one or more embodiments, the opening 138 adjacent the open end 110 of the interior wall surface 124 of the cap 102 is sized and adapted to receive a male luer connector in a press-fit connection.

Referring to Figure 5, the elastic sealing ring 126 comprises an elastomeric material.

In one or more embodiments, the elastomeric material of the elastic sealing ring 126 comprises a thermoplastic elastomer.

Referring to Figure 1 and Figures 7-8, in one or more embodiments, the locking lid 130 can engage with the engagement surface 120 to secure the elastic sealing ring 126 to the open end 110. The locking lid 130 comprises an end surface 132. The locking lid 130 prevents the elastic sealing ring 126 from moving during insertion of a male luer connector or female luer connector into the dilateable opening 128 of the elastic sealing ring 126. The locking lid 130 can be attached to cap 102 using various methods including, but not limited to, mechanical fasteners, snap-fittings, and threaded connection.

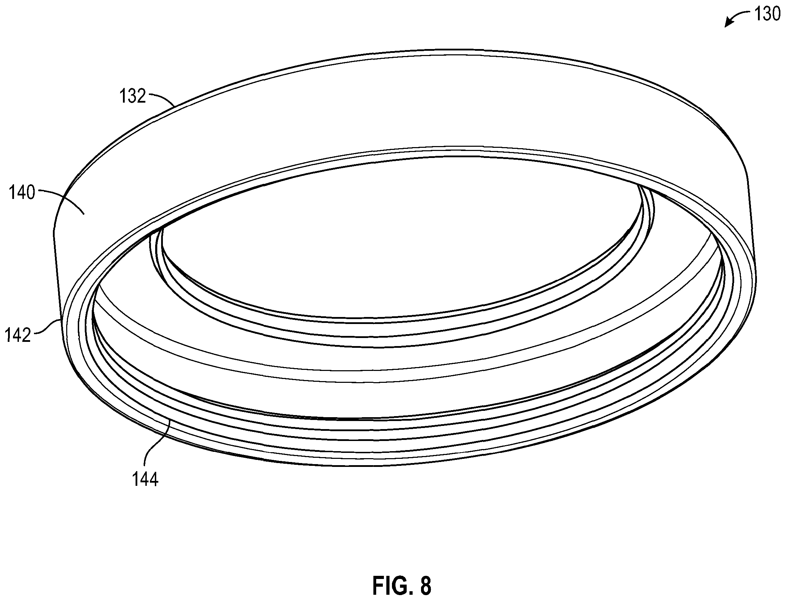

Referring to Figure 1 and Figures 7 and 8, in one or more embodiments, the locking lid 130 comprises a peripheral sidewall 140 extending substantially perpendicularly from a base 142. The locking lid 130 is adapted to be snap fit over the open end 110 of the cap 102. Referring to Figure 8, in one or more embodiments, the locking lid 130 may be connected to the cap 102 with a rim 144 disposed between the locking lid 130 and the cap 102.

In one or more alternate embodiment, an interior surface 146 of the peripheral sidewall 140 of the locking lid 130 or the exterior annular wall surface 122 of the cap 102 at the open end 110 includes at least one protrusion 148 which corresponds to at least one mating protrusion 152 disposed on an interior surface 146 of the peripheral sidewall 140 of the locking lid 130 or the exterior annular wall surface 122 of the cap 102. Protrusion 148 and mating protrusion 152 mate together such that when locking lid 130 is connected by protrusion 148 to mating protrusion 152, a leak-resistant connection is formed between the locking lid 130 and the cap 102. In one or more embodiments, protrusion 148 and mating protrusion 152 are threads.

In one or more embodiments, the locking lid 130 is a snap fit connection. In one or more embodiments, the snap fit connection comprises a protruding edge and a snap-in area. The protruding edge may be disposed on an interior surface 146 of the peripheral sidewall 140 of the locking lid 130 or the exterior annular wall surface 122 of the cap 102 at the open end 110, and the corresponding snap-in area is disposed on the opposite surface as the protruding edge to allow the protruding edge and snap-in area to interlock.

The cap 102 and the locking lid 130 are made from any of a number of types of plastic materials such as polycarbonate, polypropylene, polyethylene, polyethylene terephthalate, polylactide, acrylonitrile butadiene styrene or any other moldable plastic material used in medical devices. In one or more embodiments, the cap 102 comprises a polypropylene or polyethylene material. In one or more embodiments, the exterior wall surface 122 includes a plurality of grip members 136.

Referring to Figure 3 and Figures 9 and 10, in one or more embodiments, the absorbent material 114 is under radial compression by the internal threads to retain the absorbent material 114 in the chamber 112. In one or more embodiments, the absorbent material 114 is a nonwoven material, foam, or a sponge. In a specific embodiment, the foam is a polyurethane foam. In a specific embodiment the absorbent material 114 is in the form of a foam plug. In one or more embodiments, the absorbent material 114 includes one or more slits 154.

The device 100 can achieve disinfection when used on luer connectors by integrating disinfectant or antimicrobial agent in the chamber 112 of the cap 102. The disinfectant or antimicrobial agent can be directly included in the chamber 112 or disinfectant or antimicrobial agent can be absorbed into sponges or foam material that fills the chamber of cap 102. The device is designed to be compatible in interacting with various disinfectants. In one or more embodiments, the disinfectant or antimicrobial agent may include variations of alcohol or chlorhexidine. In one or more embodiments, the disinfectant or antimicrobial agent is selected from the group consisting essentially of isopropyl alcohol, ethanol, 2-propanol, butanol, methylparaben, ethylparaben, propylparaben, propyl gallate, butylated hydroxyanisole (BHA), butylated hydroxytoluene, t-butyl-hydroquinone, chloroxylenol, chlorohexidine, chlorhexidine diacetate, chlorohexidine gluconate, povidone iodine, alcohol, dichlorobenzyl alcohol, dehydroacetic acid, hexetidine, triclosan, hydrogen peroxide, colloidal silver, benzethonium chloride, benzalkonium chloride, octenidine, antibiotic, and mixtures thereof. In a specific embodiment, the disinfectant or antimicrobial agent comprises at least one of chlorhexidine gluconate and chlorhexidine diacetate. In one or more embodiments, the disinfectant or antimicrobial agent is a fluid or a gel.

Compression of the absorbent material 114 toward the closed end 106 of the chamber 112 upon connection to the female luer connector or the male luer connector allows the connector to contact the disinfectant or antimicrobial agent to disinfect the female luer connector or the male luer connector.

Referring to Figure 1, the peelable seal 150 on the end face 132 to prevent the disinfectant or the antimicrobial agent from exiting the chamber 112. In one or embodiments, the peelable seal 150 may be placed on the end face 132 to prevent the disinfectant or the antimicrobial agent from exiting the chamber.

In one or more embodiments, the peelable seal 150 comprises an aluminum or multi-layer polymer film peel back top. In a specific embodiment, the peelable seal 150 is heat-sealed or induction sealed to the end face of the locking lid or to the cap open end. In one or more embodiments, the peelable seal 150 comprises a moisture barrier.

The device 100 of the present disclosure is capable of continuous disinfection of a connector for 7 days. The device 100 of the present disclosure minimizes ingress of microbial agents.

In one or more embodiments, the cap exterior wall surface 122 includes a plurality of grip members 136.

Disinfecting caps currently on the market are capable of only disinfecting one of the three types of luer fitting, namely female luer of needle-free connectors, female luer of stopcocks, and male luer connectors on intravenous injection sites. Thus, to avoid having to use different types of disinfecting caps to clean different types of connectors, device 100 with cap 102 engages with male luer connectors and also with female luer connectors thereby allowing the user to clean different types of connectors with a single device. Upon mounting the cap 102 onto female luer connectors, the female luer connectors is inserted into the chamber 112 and screwed onto the threads of the cap. Upon mounting the cap onto a male luer connector, the male luer connector frictionally engage the interior wall 124 upon insertion into the chamber 112. Hence, the device of the present disclosure can be mounted onto both male and female luer connectors, thus fulfilling a current need in the art.

Other aspects of the present disclosure are directed to methods of disinfecting medical connectors and assemblies. In one or more embodiments, a method of disinfecting a medical connector comprises connecting the device of one or more embodiments to a medical connector, wherein connecting includes frictionally engaging the interior wall surface upon insertion into the chamber such that the medical connector contacts the absorbent material and the disinfectant or antimicrobial agent.

In one or more embodiments, an assembly comprises the device of one or more embodiments connected to a medical connector. In one or more embodiments, the medical connector is selected from a male luer connector, a female luer connector, and needleless connector.

Referring to Figures 11 to 14, in one or more embodiments, the cap of the device of the present disclosure may form a fluid-tight seal with a male luer connector 200, female luer connector 300, or hemodialysis connector 400. Referring to Figures 11 to 14, in one or more embodiments, the cap of the device of the present disclosure is tapered to form a fluid-tight seal with a male luer. In specific embodiments, the cap is compliant with ISO standards (e.g., ISO 594-1:1986 and ISO 594-2:1998) for forming a seal with a male luer connector.

In one or more embodiments, the cap of the device of the present disclosure has threads that have a size and pitch to engage a threadable segment of a female connector, such as for example, a female luer connector. Such connectors are generally and commonly used as catheter and other fluid-tight protective connectors in medical applications. In some embodiments, the cap provides a protective cover for a female luer connector when engaged with the connector when threads from the female luer connector engage and form a releasable connection with threads of the cap.

In some embodiments, the connector comprises a needleless injection site, which may sometimes be referred to as a needleless injection port, hub, valve, or device, or as a needleless access site, port, hub, valve, or device, and which can include such brands as, for example, Clave^{®} (available from ICU Medical, Inc.), SmartSite^{®} (available from Cardinal Health, Inc.), and Q-Syte^{™} (available from Becton, Dickinson and Company). In some embodiments, the cap can be connected with any of a variety of different needleless injection sites, such as those previously listed. In one or more embodiments, after the cap has been coupled with connector, it is unnecessary to disinfect (e.g. treat with an alcohol swab) the connector prior to each reconnection of the connector with another connector, as the connector will be kept in an uncontaminated state while coupled with the cap. Use of the cap replaces the standard swabbing protocol for cleaning connectors.

In one or more embodiments, threads of the cap are sized and pitched to engage threads of a male luer-lock connector. For example, connector can comprise the end of an IV tubing set that is disconnected from an IV catheter needleless injection site.

Reference throughout this specification to "one embodiment," "certain embodiments," "one or more embodiments" or "an embodiment" means that a particular feature, structure, material, or characteristic described in connection with the embodiment is included in at least one embodiment of the disclosure. Thus, the appearances of the phrases such as "in one or more embodiments," "in certain embodiments," "in one embodiment" or "in an embodiment" in various places throughout this specification are not necessarily referring to the same embodiment of the disclosure. Furthermore, the particular features, structures, materials, or characteristics may be combined in any suitable manner in one or more embodiments.

## Claims

1. A device for connection to a medical connector, the device comprising:
a cap (102) comprising an integral body (104), a closed end (106), an annular wall (108) having a length L_{C} extending from the closed end (106) to an open end (110) and defining a chamber (112) containing an absorbent material (114) and disinfectant or antimicrobial agent, the open end (110) having a peripheral ledge (116) extending radially inward from the open end (110) defining an end face (118) and an engagement surface (120);
the annular wall (108) having an exterior wall surface (122) and an interior wall surface (124);
an elastic sealing ring (126) having a dilatable opening (128) therethrough sized and adapted to receive a male luer connector (200), a female luer connector (300) and a hemodialysis connector (400);
**characterised in that** there is a locking lid (130) engaged with the engagement surface (120) and securing the elastic sealing ring (126) to the open end (110), the locking lid (130) having an end surface (132); and
a peelable seal (150) on the end surface (132) of the locking lid (130) to prevent the disinfectant or the antimicrobial agent from exiting the chamber (112);
wherein
the dilatable opening (128) has a diameter that is dilatable from an initial diameter in a range from about 7-9 mm to a dilated diameter in a range from about 9-12 mm.

2. The device of claim 1, wherein the female luer connector (300) is selected from the group consisting essentially of needle-free connectors, catheter luer connectors, stopcocks, and hemodialysis connector s.

3. The device of claim 1, wherein the dilatable opening (128) sized to frictionally engage a male luer connector (200).

4. The device of claim 1, wherein the male connector (200) is an intravenous tubing end, stopcock or male lock luer.

5. The device of claim 1, wherein the elastic sealing ring (126) is in contact with the peripheral ledge (116).

6. The device of claim 1, further comprising internal threads adjacent the closed end (106) that partially extend along a length of the interior wall surface (124).

7. The device of claim 1, wherein the peripheral ledge (116) is sized and adapted to receive an elastic sealing ring (126).

8. The device of claim 1, wherein the cap (102) comprises a polypropylene or polyethylene material.

9. The device of claim 1, wherein the elastic sealing ring (126) comprises an elastomeric material.

10. The device of claim 9, wherein the elastomeric material comprises a thermoplastic elastomer.

11. The device of claim 1, wherein the exterior wall surface (122) includes a plurality of grip members (136).

12. The device of claim 1, wherein the absorbent material (114) is a foam.

13. The device of claim 12, wherein the foam is a polyurethane foam.

14. The device of claim 1, wherein the absorbent material (114) is a sponge.

15. The device of claim 1, wherein the absorbent material (114) has slits.

16. The device of claim 1, wherein a compression of the absorbent material (114) toward the closed end (106) of the chamber (112) occurs upon connection to the female luer connector (300) or the male luer connector (200).

17. The device of claim 16, wherein compression of the absorbent material (114) disinfects the female luer connector (300) or the male luer connector (200).

18. The device of claim 1, wherein the absorbent material (114) is under radial compression by the internal threads to retain the absorbent material (114) in the chamber (112).

19. The device of claim 1, wherein the disinfectant or antimicrobial agent is selected from the group consisting essentially of isopropyl alcohol, ethanol, 2-propanol, butanol, methylparaben, ethylparaben, propylparaben, propyl gallate, butylated hydroxyanisole (BHA), butylated hydroxytoluene, t-butyl-hydroquinone, chloroxylenol, chlorohexidine, chlorhexidine diacetate, chlorohexidine gluconate, povidone iodine, alcohol, dichlorobenzyl alcohol, dehydroacetic acid, hexetidine, triclosan, hydrogen peroxide, colloidal silver, benzethonium chloride, benzalkonium chloride, octenidine, antibiotic, and mixtures thereof.

20. The device of claim 19, wherein the disinfectant or antimicrobial agent comprises at least one of chlorhexidine gluconate and chlorhexidine diacetate.

21. The device of claim 1, wherein the disinfectant or antimicrobial agent is a fluid or a gel.

22. The device of claim 1, wherein the peelable seal (150) comprises an aluminum or multi-layer polymer film peel back top.

23. The device of claim 1, wherein the peelable seal (150) is heat-sealed or induction sealed to the engagement surface (120).

24. A method of disinfecting a medical connector, the method comprising: connecting the device of any one of claims 1 to 23 to a medical connector, wherein connecting includes engaging the interior wall surface (124) upon insertion into the chamber (112) such that the medical connector contacts the absorbent material (114) and the disinfectant or antimicrobial agent.

25. An assembly comprising:
a connector as defined in any one of claims 1 to 23 connected with one of a male luer connector (200), a female luer connector (300), a needleless connector, and the like.

## Patentansprüche

1. Vorrichtung zur Verbindung mit einem medizinischen Konnektor, wobei die Vorrichtung aufweist:
eine Kappe (102) mit einem integralen Körper (104), einem geschlossenen Ende (106), einer ringförmigen Wand (108) mit einer Länge Lc, die sich von dem geschlossenen Ende (106) zu einem offenen Ende (110) erstreckt und eine Kammer (112) bildet, welche ein absorbierendes Material (114) und ein Desinfektionsmittel oder ein antimikrobielles Mittel enthält, wobei das offene Ende (110) einen sich radial von dem offenen Ende (110) nach innen erstreckenden umlaufenden Absatz (116) aufweist, der eine Endfläche (118) und eine Eingriffsfläche (120) bildet;
wobei die ringförmige Wand (108) eine Außenwandfläche (122) und eine Innenwandfläche (124) aufweist;
einen elastischen Dichtring (126) mit einer dort hindurch verlaufenden dehnbaren Öffnung (128), die derart bemessen und ausgebildet ist, dass sie einen männlichen Luer-Konnektor (200), einen weiblichen Luer-Konnektor (300) und einen Hämodialyse-Konnektor (400) aufnimmt;
**dadurch gekennzeichnet, dass**
ein Verriegelungsdeckel (130) vorgesehen ist, der mit der Eingriffsfläche (120) in Eingriff ist und den elastischen Dichtring (126) an dem offenen Ende (110) sichert, wobei der Verriegelungsdeckel (130) eine Endfläche (132) aufweist; und
eine abziehbare Versiegelung (150) auf der Endfläche (132) des Verriegelungsdeckels (130) vorgesehen ist, um zu verhindern, dass das Desinfektionsmittel oder das antimikrobielle Mittel aus der Kammer (112) austritt;
wobei die dehnbare Öffnung (128) einen Durchmesser aufweist, der von einem ursprünglichen Durchmesser in einem Bereich von etwa 7-9 mm zu einem gedehnten Durchmesser in einem Bereich von etwa 9-12 mm dehnbar ist.

2. Vorrichtung nach Anspruch 1, wobei der weibliche Luer-Konnektor (300) aus der Gruppe ausgewählt ist, die im Wesentlichen aus nadellosen Konnektoren, Katheter-Luer-Konnektoren, Absperrhähnen und Hämodialyse-Konnektoren besteht.

3. Vorrichtung nach Anspruch 1, wobei die dehnbare Öffnung (128) derart bemessen ist, dass sie mit einem männlichen Luer-Konnektor (200) reibschlüssig eingreift.

4. Vorrichtung nach Anspruch 1, wobei der männliche Konnektor (200) ein Ende eines intravenösen Schlauchs, ein Absperrhahn oder ein männlicher Luer-Lock ist.

5. Vorrichtung nach Anspruch 1, wobei der elastische Dichtring (126) mit dem umlaufenden Absatz (116) in Kontakt ist.

6. Vorrichtung nach Anspruch 1, die ferner Innengewinde angrenzend an das geschlossene Ende (106) aufweist, die sich teilweise entlang einer Länge der Innenwandfläche (124) erstrecken.

7. Vorrichtung nach Anspruch 1, wobei der umlaufende Absatz (116) derart bemessen und ausgebildet ist, dass er einen elastischen Dichtring (126) aufnimmt.

8. Vorrichtung nach Anspruch 1, wobei die Kappe (102) ein Polypropylen- oder Polyethylen-Material aufweist.

9. Vorrichtung nach Anspruch 1, wobei der elastische Dichtring (126) ein elastomerisches Material aufweist.

10. Vorrichtung nach Anspruch 9, wobei das elastomerische Material ein thermoplastisches Elastomer aufweist.

11. Vorrichtung nach Anspruch 1, wobei die Außenwandfläche (122) eine Vielzahl von Griffelementen (136) aufweist.

12. Vorrichtung nach Anspruch 1, wobei das absorbierende Material (114) ein Schaum ist.

13. Vorrichtung nach Anspruch 12, wobei der Schaum ein Polyurethan-Schaum ist.

14. Vorrichtung nach Anspruch 1, wobei das absorbierende Material (114) ein Schwamm ist.

15. Vorrichtung nach Anspruch 1, wobei das absorbierende Material (114) Spalte aufweist.

16. Vorrichtung nach Anspruch 1, wobei eine Kompression des absorbierenden Materials (114) in Richtung des geschlossenen Endes (106) der Kammer (112) bei Verbindung mit dem weiblichen Luer-Konnektor (300) oder dem männlichen Luer-Konnektor (200) auftritt.

17. Vorrichtung nach Anspruch 16, wobei durch die Kompression des absorbierenden Materials (114) der weibliche Luer-Konnektor (300) oder der männliche Luer-Konnektor (200) desinfiziert werden.

18. Vorrichtung nach Anspruch 1, wobei das absorbierende Material (114) durch die Innengewinde unter radialer Kompression steht, um das absorbierende Material (114) in der Kammer (112) zurückzuhalten.

19. Vorrichtung nach Anspruch 1, wobei das Desinfektionsmittel oder das antimikrobielle Mittel aus der Gruppe ausgewählt ist, die im Wesentlichen aus Folgendem besteht: Isopropylalkohol, Ethanol, 2-Propanol, Butanol, Methylparaben, Ethylparaben, Propylparaben, Propylgallat, Butylhydroxyanisol (BHA), Butylhydroxytoluen, tert-Butylhydrochinon, Chlorxylenol, Chlorhexidin, Chlorhexidindiacetat, Chlorhexidingluconat, Povidon-Jod, Alkohol, Dichlorbenzylalkohol, Dehydracetsäure, Hexetidin, Triclosan, Wasserstoffperoxid, kolloidales Silber, Benzethoniumchlorid, Benzalkoniumchlorid, Octenidin, Antibiotika, und Mischungen derselben.

20. Vorrichtung nach Anspruch 19, wobei das das Desinfektionsmittel oder das antimikrobielle Mittel Chlorhexidingluconat und/oder Chlorhexidindiacetat aufweist.

21. Vorrichtung nach Anspruch 1, wobei das Desinfektionsmittel oder das antimikrobielle Mittel ein Fluid oder ein Gel ist.

22. Vorrichtung nach Anspruch 1, wobei die abziehbare Versiegelung (150) eine Aluminium- oder mehrlagige Polymerfilm-Abziehoberseite aufweist.

23. Vorrichtung nach Anspruch 1, wobei die abziehbare Versiegelung (150) mit der Eingriffsfläche (120) warmverschweißt oder induktionsverschweißt ist.

24. Verfahren zum Desinfizieren eines medizinischen Konnektors, wobei das Verfahren umfasst: Verbinden der Vorrichtung nach einem der Ansprüche 1 bis 23 mit einem medizinischen Konnektor, wobei das Verbinden das Eingreifen mit der Innenwandfläche (124) beim Einsetzen in die Kammer (112) umfasst, so dass der medizinische Konnektor das absorbierende Material (114) und das Desinfektionsmittel oder das antimikrobielle Mittel berührt.

25. Anordnung, die aufweist:
einen Konnektor, wie nach einem der Ansprüche 1 bis 23 definiert, der mit einem männlichen Luer-Konnektor (200), einem weiblichen Luer-Konnektor (300), einem nadellosen Konnektor oder dergleichen verbunden ist.

## Revendications

1. Dispositif à raccorder à un raccord médical, le dispositif comprenant :
un capuchon (102) comprenant un corps monobloc (104), une extrémité fermée (106), une paroi annulaire (108) ayant une longueur L_{C} s'étendant de l'extrémité fermée (106) à une extrémité ouverte (110) et définissant une chambre (112) contenant un matériau absorbant (114) et un désinfectant ou un agent antimicrobien, l'extrémité ouverte (110) ayant un rebord périphérique (116) s'étendant radialement vers l'intérieur depuis l'extrémité ouverte (110) définissant une face d'extrémité (118) et une surface d'engagement (120) ;
la paroi annulaire (108) ayant une surface de paroi extérieure (122) et une surface de paroi intérieure (124) ;
une bague d'étanchéité élastique (126) ayant une ouverture dilatable (128) à travers celle-ci dimensionnée et adaptée pour recevoir un raccord luer mâle (200), un raccord luer femelle (300) et un raccord d'hémodialyse (400) ;
**caractérisé en ce qu'**il existe
un couvercle de verrouillage (130) engagé avec la surface d'engagement (120) et fixant la bague d'étanchéité élastique (126) à l'extrémité ouverte (110), le couvercle de verrouillage (130) ayant une surface d'extrémité (132) ; et
un joint d'étanchéité pelable (150) sur la surface d'extrémité (132) du couvercle de verrouillage (130) pour empêcher le désinfectant ou l'agent antimicrobien de sortir de la chambre (112) ;
dans lequel
l'ouverture dilatable (128) a un diamètre qui peut se dilater pour passer d'un diamètre initial dans une plage allant d'environ 7 à 9 mm à un diamètre dilaté dans une plage allant d'environ 9 à 12 mm.

2. Dispositif de la revendication 1, dans lequel le raccord luer femelle (300) est choisi dans le groupe constitué essentiellement par des raccords sans aiguille, des raccords luer de cathéter, des robinets d'arrêt et des raccords d'hémodialyse.

3. Dispositif de la revendication 1, dans lequel l'ouverture dilatable (128) est dimensionnée pour s'engager par frottement avec un raccord luer mâle (200).

4. Dispositif de la revendication 1, dans lequel le raccord mâle (200) est une extrémité de tubulure intraveineuse, un robinet d'arrêt ou un luer lock mâle.

5. Dispositif de la revendication 1, dans lequel la bague d'étanchéité élastique (126) est en contact avec le rebord périphérique (116).

6. Dispositif de la revendication 1, comprenant en outre des filets internes adjacents à l'extrémité fermée (106) qui s'étendent partiellement sur une longueur de la surface de paroi intérieure (124).

7. Dispositif de la revendication 1, dans lequel le rebord périphérique (116) est dimensionné et adapté pour recevoir une bague d'étanchéité élastique (126).

8. Dispositif de la revendication 1, dans lequel le capuchon (102) comprend un matériau de polypropylène ou de polyéthylène.

9. Dispositif de la revendication 1, dans lequel la bague d'étanchéité élastique (126) comprend un matériau élastomère.

10. Dispositif de la revendication 9, dans lequel le matériau élastomère comprend un élastomère thermoplastique.

11. Dispositif de la revendication 1, dans lequel la surface de paroi extérieure (122) comprend une pluralité d'éléments de préhension (136).

12. Dispositif de la revendication 1, dans lequel le matériau absorbant (114) est une mousse.

13. Dispositif de la revendication 12, dans lequel la mousse est une mousse de polyuréthanne.

14. Dispositif de la revendication 1, dans lequel le matériau absorbant (114) est une éponge.

15. Dispositif de la revendication 1, dans lequel le matériau absorbant (114) a des fentes.

16. Dispositif de la revendication 1, dans lequel une compression du matériau absorbant (114) vers l'extrémité fermée (106) de la chambre (112) se produit lors du raccordement au raccord luer femelle (300) ou au raccord luer mâle (200).

17. Dispositif de la revendication 16, dans lequel la compression du matériau absorbant (114) désinfecte le raccord luer femelle (300) ou le raccord luer mâle (200).

18. Dispositif de la revendication 1, dans lequel le matériau absorbant (114) est sous compression radiale par les filets internes pour retenir le matériau absorbant (114) dans la chambre (112).

19. Dispositif de la revendication 1, dans lequel le désinfectant ou l'agent antimicrobien est choisi dans le groupe constitué essentiellement par l'alcool isopropylique, l'éthanol, le 2-propanol, le butanol, le méthylparabène, l'éthylparabène, le propylparabène, le gallate de propyle, l'hydroxyanisole butylé (BHA), l'hydroxytoluène butylé, la t-butyl-hydroquinone, le chloroxylénol, la chlorhexidine, le diacétate de chlorhexidine, le gluconate de chlorhexidine, la povidone iodée, un alcool, l'alcool dichlorobenzylique, l'acide déhydroacétique, l'hexétidine, le triclosan, le peroxyde d'hydrogène, l'argent colloïdal, le chlorure de benzéthonium, le chlorure de benzalkonium, l'octénidine, un antibiotique et leurs mélanges.

20. Dispositif de la revendication 19, dans lequel le désinfectant ou l'agent antimicrobien comprend au moins l'un parmi le gluconate de chlorhexidine et le diacétate de chlorhexidine.

21. Dispositif de la revendication 1, dans lequel le désinfectant ou l'agent antimicrobien est un fluide ou un gel.

22. Dispositif de la revendication 1, dans lequel le joint d'étanchéité pelable (150) comprend un dessus pelable en film polymère multicouche ou en aluminium.

23. Dispositif de la revendication 1, dans lequel le joint d'étanchéité pelable (150) est thermoscellé ou scellé par induction à la surface d'engagement (120).

24. Procédé de désinfection d'un raccord médical, le procédé comprenant : le raccordement du dispositif de l'une quelconque des revendications 1 à 23 à un raccord médical, dans lequel le raccordement comprend l'engagement de la surface de paroi intérieure (124) lors de l'insertion dans la chambre (112) de sorte le raccord médical entre en contact avec le matériau absorbant (114) et le désinfectant ou l'agent antimicrobien.

25. Ensemble comprenant :
un raccord tel que défini dans l'une quelconque des revendications 1 à 23 raccordé à l'un parmi un raccord luer mâle (200), un raccord luer femelle (300), un raccord sans aiguille, et autres analogues.
